(19) Europäisches Patentamt
European Patent Office
Office européen des brevets

(11) Publication number: 0 384 392 A1

(12) # EUROPEAN PATENT APPLICATION

(21) Application number: 90103235.9

(51) Int. Cl.[5]: C07C 255/50, C07C 253/14

(22) Date of filing: 20.02.90

(30) Priority: 21.02.89 US 312164

(43) Date of publication of application: 29.08.90 Bulletin 90/35

(84) Designated Contracting States: BE CH DE FR GB IT LI NL

(71) Applicant: OCCIDENTAL CHEMICAL CORPORATION
P.O. Box 189
Niagara Falls New York 14302(US)

(72) Inventor: Maul, James J.
15 Beaver Lane
Grand Island, N.Y. 14072(US)
Inventor: Seper, Karl W.
2043 Whitehaven Road, No. 5
Grand Island, N.Y. 14072(US)
Inventor: Henry C., Lin
1971 Huth Road
Grand Island, N.Y. 14072(US)

(74) Representative: Vossius & Partner
Siebertstrasse 4 P.O. Box 86 07 67
D-8000 München 86(DE)

(54) **Cyanation of haloaromatics utilizing catalysts generated in situ starting with NiCl2 or NiCl2 6H2O.**

(57) Aromatic halide is converted to aromatic nitrile, e.g., p-chlorobenzotrifluoride is converted to 4-(trifluoromethyl)benzonitrile, utilizing $Ni(P\phi_3)_3$ as a catalyst where said catalyst is formed starting with $NiCl_2$ or $NiCl_2 \cdot 6H_2O$ where formation of said catalyst starting with $NiCl_2$ or $NiCl_2 \cdot 6H_2O$ and conversion of halide to nitrile are carried out in a single reactor vessel. The method comprises the steps of (a) forming catalyst by steps comprising (i) forming essentially dry $Ni(P\phi_3)Cl_2$ in situ in said reactor vessel starting with $NiCl_2$ or $NiCl_2 \cdot 6H_2O$, (ii) reacting said essentially dry $Ni(P\phi_3)_2Cl_2$ with $P\phi_3$ in the presence of a reducing metal in $C_2$-$C_5$ alcohol or aprotic polar reaction solvent in said reactor vessel to produce $Ni(P\phi_3)_3$ catalyst therein, and (b) adding said halide into said reactor vessel to form a complex in $C_2$-$C_5$ alcohol or aprotic polar reaction solvent from said halide and said catalyst and adding alkali metal cyanide to convert halogen to CN and thereby produce nitrile. When anhydrous $NiCl_2$ is a starting material, steps (a)(i) and (a)(ii) are preferably carried out as a single step where anhydrous $NiCl_2$ and at least three equivalents of $P\phi_3$ are added to $C_2$-$C_5$ alcohol or aprotic polar solvent followed by addition of reducing metal powder and reaction is carried out to convert $NiCl_2$ to $Ni(P\phi_3)_3$ with intermediate formation of $Ni(P\phi_3)_2Cl_2$. When $NiCl_2 \cdot 6H_2O$ is a starting material, essentially dry $Ni(P\phi_3)_2Cl_2$ is formed in step (a)(i) by forming an admixture thereof with azeotrope-forming liquid (e.g., anhydrous $C_2$-$C_5$ alcohol or aprotic polar solvent or toluene) and heating to distill off water and forming essentially dry $NiCl_2$ which is converted to essentially dry $Ni(P\phi_3)_2Cl_2$ or the $NiCl_2 \cdot 6H_2O$ is reacted with $P\phi_3$ to form wet $Ni(P\phi_3)_2Cl_2$ which is dried by forming an azeotropic mixture and distilling to remove water or by heating or the $NiCl_2 \cdot 6H_2O$ is made essentially dry by heating with or without $P\phi_3$ present in the absence of reaction solvent to flash off water and form essentially dry $NiCl_2$ which is reacted with $P\phi_3$. The preferred $C_2$-$C_5$ alcohol is t-butanol which minimizes formation of hydrodehalogenation side-product in step (b) compared to other alcohols. The preferred aprotic polar solvent is acetonitrile.

EP 0 384 392 A1

# CYANATION OF HALOAROMATICS UTILIZING CATALYSTS GENERATED IN SITU STARTING WITH $NiCl_2$ or $NiCl_2 \cdot 6H_2O$

This invention is directed to converting aromatic halides to aromatic nitriles, e.g., to converting p-chlorobenzotrifluoride to 4-(trifluoromethyl)benzonitrile. The products are useful, e.g., as chemical intermediates for production of pharmaceuticals and agricultural chemicals.

Davison et al U.S. Patent No. 4,499,025 teaches converting aryl halides including p-chlorobenzotrifluoride to corresponding nitriles utilizing a zero valent Group VIII catalyst which can be $Ni(P\phi_3)_3$ and a cyanide ion source which can be alkali metal cyanide in a solvent which can be an alcohol or an aprotic polar solvent.

Cassar et al, Advances in Chemistry Series #132, pp. 252-273 (1974) discloses a one pot reaction where catalyst is formed in situ from $Ni(P\phi_3)_2Cl_2$, powdered manganese-iron alloy, and triphenylphosphine in ethanol and then aryl halide (1-chloronaphthalene) is added and then NaCN is added to form nitrile (1-cyanonaphthalene).

The problem with starting with either $Ni(P\phi_3)_3$ or $Ni(P\phi_3)_2Cl_2$ as described above is that these are very expensive materials and therefore are inappropriate for a commercially viable process.

One object herein is to lower the cost of catalyst thereby providing a commercially more attractive process.

Another problem in this field is eliminating formation of reduction product (hydrodehalogenation) where halogen on the aromatic ring is replaced by hydrogen, i.e., eliminating conversion of Ar-X to Ar-H.

Use of methanol as a reaction solvent provides insignificant percentage conversion to nitrile. See the aforementioned Cassar et al article at page 260.

Moreover, the use of ethanol as a reaction solvent as is disclosed in Davison et al and Cassar et al discussed above has been found to provide hydrodehalogenation product (i.e., where hydrogen rather than CN replaces halogen) in the instant process in excess of 5%, normally 5-25%. Isopropanol has also been found to provide hydrodehalogenation product in the 5-25% range.

In a preferred embodiment herein, an object is to increase the percentage of nitrile product obtained compared to where methanol or ethanol or propanols are used.

It has been discovered herein that the negative provided by the high cost of $Ni(P\phi_3)_3$ and $Ni(P\phi_3)_2Cl_2$ can be overcome by forming $Ni(P\phi_3)_3$ in situ starting with $NiCl_2$ or $NiCl_2 \cdot 6H_2O$ whereby catalyst formation from $NiCl_2$ or $NiCl_2 \cdot 6H_2O$ and cyanation reaction are carried out in a single reactor vessel, i.e., in a one pot system. While it is known to prepare $Ni(P\phi_3)_3$ from anhydrous nickel chloride (See Tolman, C.A., et al, Journal of the American Chemical Society, 94:8, 4/19/72, pp. 2669-2676 at page 2671) and it is known to prepare anhydrous $Ni(P\phi_3)_2Cl_2$ from anhydrcus $NiCl_2$ (Thabet, S.K., et al, Inorg. Nucl. Chem. Letters, 8(2), 211) and from $NiCl_2$ $6H_2O$ (Itatani, H., et al, J. Am. Chem. Soc. 89:7, 3/29/67, pp. 1600-1602), in each case the product was isolated, and there is no disclosure of preparation thereof in the same reaction vessel as is used for cyanation to thereby reduce processing costs.

It has further been discovered herein that extent of hydrodehalogenation in the cyanation reaction is related to the reaction solvent used and to chain length and branching when alcohol reaction solvent is used and that increased branching and chain length in an alcohol reaction solvent leads to decreased hydrodehalogenation and that anhydrous t-butanol is a preferred reaction solvent for cyanation.

It has further been discovered herein that essentially dry aprotic polar solvents are very preferred solvents for the cyanation reaction. This is because, it has been discovered that when these are utilized, side reactions such as hydrodehalogenation and coupling are minimized, and the reaction time for cyanation is usually less than when alcohol reaction solvents are used.

Thus, the present invention is directed to a method of converting aromatic halide to aromatic nitrile utilizing $Ni(P\phi_3)_3$ as a catalyst, where said catalyst is formed starting with $NiCl_2$ or $NiCl_2 \cdot 6H_2O$ and where formation of said catalyst starting with $NiCl_2$ or $NiCl_2 \cdot 6H_2O$ and conversion of halide to a nitrile are carried out in a single reactor vessel, said aromatic halide having the formula:

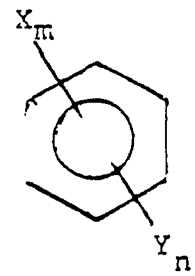

where X is Cl or Br, Y is CHO or $CF_3$, m ranges from 1 to 3, and n ranges from 0 to 2, said method comprising the steps of:

(a) forming said catalyst by steps comprising:

(i) forming essentially dry $Ni(P\phi_3)_2Cl_2$ in situ in said reactor vessel starting with $NiCl_2$ or $NiCl_2 \cdot 6H_2O$ and at least two equivalents of $P\phi_3$;

(ii) reacting said $Ni(P\phi_3)_2Cl_2$ with at least one equivalent of $P\phi_3$ in the presence of a reducing metal with a greater reduction potential than nickel in a reaction solvent consisting of a $C_2$-$C_5$ alcohol or an aprotic polar solvent in said reactor vessel to produce $Ni(P\phi_3)_3$ catalyst therein;

(b) adding said aromatic halide to said reactor vessel to form a complex in reaction solvent from said aromatic halide and said catalyst and adding an alkali metal cyanide preferably NaCN or KCN into said reactor vessel to convert X to CN (i.e., to convert all the X's which are present to CN) and thereby produce nitrile product.

In a preferred embodiment, the halide is p-chlorobenzotrifluoride and the nitrile which is formed is 4-(trifluoromethyl)benzonitrile.

In a preferred embodiment the reaction solvent in steps (a)(ii) and (b) is a $C_4$ alcohol, very preferably t-butanol, and step (a)(i) involves forming essentially dry $Ni(P\phi_3)_2Cl_2$ starting with $NiCl_2 \cdot 6H_2O$ by steps comprising forming a slurry of $NiCl_2 \cdot 6H_2O$ in said alcohol and heating to cause conversion of the hydrate to essentially dry $NiCl_2$ and to distill off the water which is present as an azeotrope and sufficient of said alcohol for distilling off of the water.

In a more preferred embodiment the reaction solvent in steps (a)(ii) and (b) is an aprotic polar solvent, very preferably acetonitrile, and step (a)(i) involves forming essentially dry $Ni(P\phi_3)_2Cl_2$ starting with $NiCl_2 \cdot 6H_2O$ by steps comprising forming a slurry of $NiCl_2 \cdot 6H_2O$ in acetonitrile and heating to cause conversion of the hydrate to essentially dry $NiCl_2$ and to distill off the water which is present as an azeotrope and sufficient of said acetonitrile for distilling off the water.

The symbol $\phi$ is used herein to represent the phenyl radical, i.e., $C_6H_5$-.

The term "essentially dry" is used herein to mean as follows: In step (a)(i), essentially dry $NiCl_2$ is yellow in color and contains less than about two (2) water of hydration. In steps (a)(i) and (a)(ii), essentially dry $Ni(P\phi_3)_2Cl_2$ contains less than about 8% $H_2O$ by weight. In step (b) essentially dry reaction mixture contains less than about 0.5% $H_2O$ expressed as weight percent of solvent present. In steps (a)(i), (a)(ii) and (b), essentially dry solvent contains less than about 0.5% $H_2O$ expressed as weight percent of solvent present.

The aromatic halide starting materials include, for example, p-chlorobenzotrifluoride which is a preferred starting material, p-bromobenzotrifluoride,m-chlorobenzotrifluoride, m-bromobenzotrifluoride,p-chlorobenzaldehyde, p-bromobenzaldehyde,m-chlorobenzaldehyde, m-bromobenzaldehyde,chlorobenzene,bromobenzene, 1,2-dichlorobenzene, 1,3-dichlorobenzene, 1,4-dichlorobenzene, 1,2,3-trichlorobenzene, 1,2,4-trichlorobenzene, 1,3,5-trichlorobenzene, 3,4-dichlorobenzene, 3,4-dibromobenzaldehyde, 3,4-dichlorobenzotrifluoride, 3,4,5-trichlorobenzotrifluoride, 1,2-ditrifluoromethyl-4-chlorobenzene, 1,3-ditrifluoromethyl-5-chlorobenzene, 1,2-ditrifluoromethyl-4-bromobenzene, 1,3-ditrifluoromethyl-5-bromobenzene, 1,2-diformyl-4-chlorobenzene, 1,3-diformyl-5-chlorobenzene, 1,2-diformyl-4-bromobenzene and 1,3-diformyl-5-bromobenzene.

The aromatic nitrile product corresponds to the aromatic halide starting material with the halide(s) replaced with CN. Thus, for example, p-chlorobenzotrifluoride is converted to 4-(trifluoromethyl)benzonitrile by the process herein and chlorobenzene is converted to benzonitrile by the process herein.

We turn now to step (a)(i). This is readily carried out by starting with anhydrous $NiCl_2$ and reacting with two equivalents of $P\phi_3$ in anhydrous reaction solvent selected from the group consisting of $C_2$-$C_5$ alcohols and aprotic polar solvents. Suitable alcohols include, for example, ethanol, isopropanol, isobutanol, t-butanol, 1-pentanol, 2-pentanol and 3-pentanol. The preferred alcohols are $C_4$ alcohols, very preferably t-butanol since it is a preferred reaction solvent in step (b), and when the t-butanol used in this step, it preferably is

carried over into succeeding steps. Suitable aprotic polar solvents include, for example, acetonitrile, dimethylformamide, and tetrahydrofuran. The preferred aprotic polar solvents are those which boil lower than 110 °C, very preferably acetonitrile which is the preferred reaction solvent for steps (a)(ii) and (b) and is preferably carried over into these steps. The reaction is readily carried out at temperatures from 25 °C to reflux temperature. For alcohol reaction solvents, reflux temperature is preferred and the the time of reaction at reflux temperature is normally 0.5 to 3 hours. The preferred temperature range where aprotic polar solvents are used is 25 °C to 60 °C and at these temperatures the time of reaction is normally 0.5 to 3 hours.

Step (a)(1) is also readily carried out starting with $NiCl_2 \cdot 6H_2O$ or moisture containing $NiCl_2$, hereinafter collectively referred to as wet $NiCl_2$.

In these cases, the wet $NiCl_2$ is (1) either made essentially dry by forming an azeotrope mixture and distilling to remove the water and form essentially dry $NiCl_2$ or (2) the wet $NiCl_2$ is reacted with 2 equivalents of $P\phi_3$ to form $Ni(P\phi_3)_2Cl_2$ which is dried by forming an azeotropic mixture and distilling to remove water or by heating to form essentially dry $Ni(P\phi_3)_2Cl_2$ or (3) the wet $NiCl_2$ is made essentially dry by heating with or without $P\phi_3$ being present in the absence of the reaction solvent to remove water prior to reaction with $P\phi_3$.

Turning to the embodiment where step (a)(i) involves starting with wet $NiCl_2$ and predrying by forming an azeotropic mixture and distilling, the essentially dry $Ni(P\phi_3)_2Cl_2$ is formed by steps comprising starting with wet $NiCl_2$ and forming an admixture of wet $NiCl_2$ in a liquid which forms an azeotropic mixture with water (hereinafter "azeotrope-forming liquid") and has a boiling point at least 10 degrees less than that of the ultimate nitrile product (so as to allow easy separation by distillation on carryover of said azeotrope-forming liquid into step (a)(ii) and from there into step (b)), and heating to remove sufficient of the water of hydration and to distill off sufficient water and sufficient azeotrope-forming liquid so as not to significantly interfere with reaction in steps (a)(ii) and (b), and reacting the formed essentially dry $NiCl_2$ with two equivalents of $P\phi_3$ in $C_2$-$C_5$ alcohol or aprotic polar reaction solvent to form essentially dry $Ni(P\phi_3)_2Cl_2$. Once essentially dry $NiCl_2$ is formed, the conditions to form essentially dry $Ni(P\phi_3)_2Cl_2$ are the same as those described above for formation of $Ni(P\phi_3)_2Cl_2$ from anhydrous $NiCl_2$.

Turning to the embodiment of step (a)(i) where wet $NiCl_2$ is reacted with two equivalents of $P\phi_3$ to form wet $Ni(P\phi_3)_2Cl_2$ which is dried by heating or by forming an azeotropic mixture and distilling, the reaction is carried out, for example, by heating the wet $NiCl_2$ with at least two equivalents of $P\phi_3$ under an inert (e.g. nitrogen) atmosphere at 40 to 100 °C for 0.5 to 3 hours to form wet $Ni(P\phi_3)_2Cl_2$ and drying is carried out by heating at 60 to 100 °C in the absence of solvent or by heating an admixture of the wet $Ni(P\phi_3)_2Cl_2$ with liquid which forms an azeotrope with water and has a boiling point of at least 10 °C less than the ultimate nitrile product to remove sufficient water and sufficient azeotrope-forming liquid to form essentially dry $Ni(P\phi_3)_2Cl_2$ and so as not to significantly interfere with reaction in steps (a)(ii) and (b).

Whether wet $NiCl_2$ is dried utilizing an azeotrope forming liquid or wet $Ni(P\phi_3)_2Cl_2$ is dried utilizing an azeotrope forming liquid, the azeotrope-forming liquid is preferably a $C_2$-$C_5$ alcohol (e.g., those named specifically above) or an aprotic polar solvent (e.g., those specifically named above) since there can be carryover of said liquid to succeeding steps (a)(ii) and (b) where these are important. Azeotrope-forming liquid can also be one which is not an alcohol or aprotic polar solvent such as, zylene or benzene, but care should be taken to distill off essentially all of such prior to step (a)(ii) since carryover to step (a)(ii) is undesirable. Preferably azeotrope-forming liquid for step (a)(i) is a $C_4$ alcohol, very preferably t-butanol, or more preferably an aprotic polar solvent, most preferably acetonitrile since these are preferred reaction solvents in step (b) and carryover to succeeding steps simply supplies reaction solvent for step (b).

Turning now to the embodiment where step (a)(i) involves starting with wet $NiCl_2$ and drying in the absence of solvent and $P\phi_3$, said drying is readily carried out by heating under vacuum at temperatures ranging from 40 to 85 °C for 1 to 5 hours to form essentially dry $NiCl_2$. Once essentially dry $NiCl_2$ is formed and the water is removed, the conditions to form essentially dry $Ni(P\phi_3)_2Cl_2$ are the same as those described above for formation of essentially dry $Ni(P\phi_3)_2Cl_2$ from anhydrous $NiCl_2$.

Turning now to the embodiment where step (a)(i) involves starting with wet $NiCl_2$ and drying and reacting in the absence of solvent, essentially dry $Ni(P\phi_3)_3Cl_2$ is formed by steps comprising starting with wet $NiCl_2$ and heating said wet $NiCl_2$ with at least two equivalents of $P\phi_3$ in the absence of solvent to flash off water and react $NiCl_2$ with two equivalents of $P\phi_3$. This step is readily carried out by heating at 80-120 °C for 0.5 to 1 hour.

We turn now to the step (a)(ii). The reducing metal is preferably used in powdered form and is selected from the group consisting of metals with greater reduction potential than nickel, for example, zinc, magnesium, manganese or manganese-iron alloy. It is normally used in an amount such that the molar ratio of reducing metal to $NiCl_2$ used to form $Ni(P\phi_3)_2Cl_2$ ranges from 1 to 4. In this step the reaction solvent can

be, for example, the same as those specifically recited above in relation to step (a)(i). The preferred alcohol reaction solvent for this step is t-butanol since this alcohol reaction solvent is a preferred one for step (b) and there is carryover of reaction solvent from step (a)(ii) to step (b). The aprotic polar solvents are the same as those described in conjunction with step (a)(i). The preferred aprotic polar solvent for step (a)(ii) is acetonitrile since this reaction solvent is a preferred reaction solvent for step (b) and there is carryover of reaction solvent from step (a)(ii) to step (b). Step (a)(ii) is readily carried out at a temperature ranging from 25° C to reflux temperature. For alcohol reaction solvents, reflux temperature is preferred and the reaction time at reflux temperature is normally 0.5 to 3 hours. For aprotic polar solvents, a reaction temperature ranging from 25° C to 60° C is preferred, and at these temperatures the time of reaction is normally 0.5 to 3 hours. Formation of $Ni(P\phi_3)_3$ is signaled by a blood red color.

Once essentially dry $NiCl_2$ is present (e.g. if anhydrous $NiCl_2$ is purchased and used as a starting material), steps (a)(i) and (a)(ii) may be carried out as a single step where essentially dry $NiCl_2$ and at least three equivalents of $P\phi_3$ are added to reaction solvent ($C_2$-$C_5$ alcohol or aprotic polar reaction solvent) followed by addition of reducing metal and reaction to convert the $NiCl_2$ to $Ni(P\phi_3)_3$ with intermediate formation of $Ni(P\phi_3)_2Cl_2$. The conversion of $NiCl_2$ to $Ni(P\phi_3)_3$ is readily carried out at a temperature ranging from 40° C to reflux with reflux temperature preferred in alcohol solvent. In polar aprotic solvents about 40° C is preferred. The conversion of essentially dry $NiCl_2$ to $Ni(P\phi_3)_3$ is readily obtained in 2 to 6 hours.

Turning now to step (b), the $C_2$-$C_5$ alcohol or aprotic polar reaction solvent from step (a)(ii) carries over to be the $C_2$-$C_5$ alcohol or aprotic polar reaction solvent for this step and more can be added for step (b) if this is considered appropriate. The aromatic halide is added, for example, over a period of 5 minutes to 1 hour depending on the scale of the preparation, while the temperature of the solution is at 40° C to reflux. On addition of the halide, a reactive complex is formed from the halide and the $Ni(P\phi_3)_3$ catalyst in solution in the alcohol reaction solvent or the aprotic polar reaction solvent, which is denoted by color change from blood red to green. The alkali metal cyanide is added in at least a stoichiometric amount, e.g., preferably in a molar ratio of alkali metal cyanide to organic halide where m equals 1 of about 1:1, preferably to reaction solution which is below reflux to mitigate the possibility of loss of said cyanide reactant. Reaction is readily carried out in the range of 40° C to reflux temperature over a period of about 5 to about 18 hours. In the instances where the reaction solvent is an aprotic polar solvent, temperatures in the range of 40 to 60° C are usually preferred. In other cases reflux temperature is preferred to mitigate the need for temperature control. The reaction mixture in this step should be essentially dry to minimize hydrodehalogenation and decomposition of nickel catalyst complexes to inactive forms.

Stoichiometric quantities of reactants can be used in all steps herein. For $P\phi_3$ an excess of about 33% is advantageous. Quantities in excess of twice stoichiometric are considered undesirable as wasting reactant.

In all steps herein it is important to minimize the presence of water in the reaction system since carryover of water to step (b) fosters hydrodehalogenation (i.e., replacement of halogen with H rather than CN). Water also promotes decomposition of Ni catalyst complexes to inactive forms. Step (a)(i) can initially have water present since wet nickel chloride can be the reactant. In such cases where water is introduced by use of wet $NiCl_2$ starting material, the wet $NiCl_2$ is converted to essentially dry $NiCl_2$ or essentially dry $Ni(P\phi_3)_2Cl_2$ for or during step (a)(i). In line with the importance of minimizing the presence of water, it is preferred that any azeotrope-forming liquid or reaction solvent should be essentially dry at the completion of step (a)(i) and that all reaction solvents should be essentially dry for steps (a)(ii) and (b); this means that the azeotrope-forming liquids and reaction solvents should contain less than about 0.5% by weight of water. All steps should be carried out in a dry inert atmosphere, e.g., under a nitrogen atmosphere.

The nitrile product is readily recovered from the admixture in the reactor by filtering cool admixture (e.g., at room temperature through anhydrous potassium carbonate) and distilling. Distillation and recovering a cut at 85-95° C provides suitable isolation when 4-(trifluoromethyl)benzonitrile is the product.

It is important that the reaction solvent in step (b) be a $C_2$-$C_5$ alcohol or an aprotic polar solvent. To eliminate the need for replacing reaction solvent between steps (a) and (b), the reaction solvent in step (a)-(ii) should be $C_2$-$C_5$ alcohol or an aprotic polar solvent and once essentially dry $NiCl_2$ is present in the system, catalyst formation and cyanation reaction can be carried out in the same reaction solvent. The use of methanol rather than $C_2$-$C_5$ alcohol is not permitted in steps (a)(ii) and (b) since methanol fosters hydrodehalogenation and hydrodehalogenation product is the major by-product. In addition, the catalyst life in step (b) is usually very short if methanol is used as solvent. The use of alcohols greater than $C_5$ is satisfactory for cyanation but causes recovery problems since the boiling point of the alcohols is similar to that of the nitrile product. The uses of higher boiling point aprotic polar solvents is also satisfactory for cyanation but likewise causes recovery problems since their boiling point is equal or greater than that of the nitrile product. Thus, usually the aprotic polar solvent for step (b) as well as for prior steps should boil lower

than about 110° C.

As indicated above, higher molecular weight and branching in the alcohol reaction solvent foster cyanation and broadly a decrease in the percentage of hydrodehalogenation product. Percentage hydrodehalogenation product as a function of reaction solvent in step (b) has been found to be as follows in terms of mole % of starting aromatic halide:

| Reaction Solvent | Percentage Hydrodehalogenation Product |
|---|---|
| Ethanol | 5-25% |
| Isopropanol | 5-25% |
| Isobutanol | 3-5% |
| t-Butanol | 1-2% |

As indicated above, t-butanol is preferred as an alcohol reaction solvent; its use gives very low percentage hydrodehalogenation product while providing boiling point difference from nitrile product to maximize ease of recovery of nitrile product. While the inventors do not wish to be held to a specific theoretical explanation, it is felt that improved performance of the more highly branched alcohols may be broadly correlated to the lower acidity of the more highly branched alcohols.

The use of aprotic polar solvents are even more preferred since hydrodehalogenation is further minimized when they are used. As shown in Example IX below, the percentage of hydrodehalogenation is essentially less than 1% in acetonitrile solvent. This is consistent with the lack of acidic protons in aprotic polar solvents.

Aprotic polar solvents are also even more preferred since it has been discovered that step (b) is usually completed faster in aprotic polar solvents (compare Examples I and IX below).

The process herein allows starting with inexpensive materials, i.e., $NiCl_2$ and $NiCl_2 \cdot 6H_2O$ and $P\phi_3$ rather than relatively expensive $Ni(P\phi_3)_2Cl_2$ or $Ni(P\phi_3)_3$ and proceeding from said inexpensive materials without intermediate separation steps while mitigating the chance for entry of excess water and air into the cyanation step, thereby for the first time providing a commercially viable process for converting aromatic halides to aromatic nitriles.

The invention herein is illustrated by the following examples:

## Example I

To a 3-neck 500 mL flask equipped with a reflux condenser, pressure equalizing addition funnel and rubber septum was added under nitrogen 40 mL of anhydrous isopropyl alcohol. To the addition funnel was charged p-chlorobenzotrifluoride (PCBTF) (10.6g, 60 mmol). To this magnetically stirred solution was then added in order, anhydrous $NiCl_2$ (0.14g, 1.1 mmol), triphenylphosphine (1.05g, 4 mmol), and powdered zinc (0.41g, 5 mmol). Under nitrogen the stirred solution was brought to reflux for a period of 2 hours when the color of the reaction solvent turned blood red. To the red solution was then added the p-chlorobenzotrifluoride over a period of about 5 minutes during which time the solution turned green in color. The mixture was allowed to cool below reflux temperature and sodium cyanide (2.94g, 60 mmol) was added in one portion via a port fitted with the rubber stopper. The reaction was brought to reflux and monitored by gas chromatography. The reaction was complete in about 18 hours. The extent of hydrodehalogenation product, i.e., trifluoromethylbenzene in said reaction mixture was about 20% of p-chlorobenzotrifluoride reacted versus 80% for 4-(trifluoromethyl)benzonitrile. The reaction mixture was filtered through anhydrous potassium carbonate to yield an orange/yellow solution which was distilled through a 6 inch packed column: The desired 4-(trifluoromethyl)benzonitrile distilled at 93-95° C/30 mm.

## Example II

When ethanol was substituted for the isopropyl alcohol in the above Example I, 4-(trifluoromethyl)-

benzonitrile was still the major product but the amount of hydrodehalogenation product was about 8% of the moles of p-chlorobenzotrifluoride reacted.

Example III

When isobutanol was substituted for the isopropanol in the above Example I, the reaction mixture was heated to 80 °C rather than at reflux during cyanation. 4-(trifluoromethyl)benzonitrile was still the major product but the amount of trifluoromethyl-benzene hydrodehalogenation product was only about 5% of the moles of p-chlorobenzotrifluoride reacted.

Example IV

To a 3-neck 1L flask equipped with a reflux condenser, pressure equalizing addition funnel and rubber septum was added under nitrogen 160 mL of anhydrous t-butanol. To the t-butanol with magnetic stirring was then added, in order, anhydrous $NiCl_2$ (0.56g, 4.3 mmol), triphenylphosphine (4.44g, 17 mmol), and powdered zinc (0.8g, 13 mmol). The solution was stirred at reflux for two hours when the color of the reaction mixture became blood red. To the solution was then added p-chlorobenzotrifluoride (42.6g, 240 mmoles) over a period of about 5 minutes at which time the solution turned green. The reaction mixture was partially cooled and then sodium cyanide (11.6g, 240 mmoles) was added in one portion. The reaction was brought to reflux and monitored via GLC. After about 18 hours the reaction was essentially complete. GLC indicated that about 1% of the reacted p-chlorobenzotrifluoride has undergone hydrodehalogenation side reaction. The mixture was cooled, filtered through potassium carbonate and distilled as described in Example I. The boiling point of the desired 4-(trifluoromethyl)benzonitrile was 93-95 °C/35 mm.

When in the above Example IV an equimolar amount of KCN was substituted for the NaCN, 4-(trifluoromethyl)benzonitrile is still the major product and hydrodehalogenation occurred to an extent of about 1%.

When in the above Example IV an equimolar amount of manganese is substituted for the zinc, 4-(trifluoromethyl)benzonitrile is still the major product and hydrodechlorination occurs to the extent of less than about 5%.

When in the above Example IV an equimolar amount of p-bromobenzotrifluoride is substituted for the p-chlorobenzotrifluoride, 4-(trifluoromethyl)benzonitrile is the major product and hydrodehalogenation occurs to an extent of about 1%.

When in the above Example IV, an equimolar amount of p-chlorobenzaldehyde was substituted for the p-chlorobenzonitrile, the major product was 4-cyanobenzaldehyde.

When in the above Example IV, an equimolar amount of m-chlorobenzotrifluoride was substituted for the p-chlorobenzotrifluoride, the major product was 3-(trifluoromethyl)benzonitrile.

When in the above Example IV, an equimolar amount of chlorobenzene was substituted for the p-chlorobenzotrifluoride, the major product was benzonitrile.

Example V

When in the above Example IV, an equal amount of acetonitrile is substituted for the t-butanol and the temperature used is 45 °C and the cyanation is carried out over a period of 5 hours, 4-(trifluoromethyl)-benzonitrile is still the major product and less than 1% hydrodehalogenation occurs.

When in the above Example V, tetrahydrofuran is substituted for the acetonitrile, 4-(trifluoromethyl)-benzonitrile is still the major product and less than 1% hydrodehalogenation occurs.

Example VI

To a 3-neck flask equipped with a reflux condenser, pressure equalizing addition funnel, distillation head

and nitrogen protection system was added 20 mL of anhydrous t-butanol and 0.96g (4.2 mmoles) of $NiCl_2 \cdot 6H_2O$. The slurry was heated to 50 °C at which time the green hexahydrate was observed to form yellow essentially dry nickel chloride. The temperature was raised to 90 °C and the wet t-butanol azeotrope was distilled away. To the cooled residue was added 4.2g, 0.18 mmol of triphenylphosphine and 30 ml dry t-butanol. Heating for 2 hours resulted in the formation of $Ni(P\phi_3)_2Cl_2$. To the mixture was added an additional 140 ml dry t-butanol and 0.56g, 8.6 mmol of powdered zinc. The reaction mixture was heated at reflux for 2 hours when the color of the reaction mixture turned to blood red as $Ni(P\phi_3)_3$ was formed. To the reaction mixture was added 42.6g, 240 mmole of p-chlorobenzotrifluoride followed by 11.5g, 240 mmole sodium cyanide. The reaction was monitored via GLC which indicated that reaction was complete in about 24 hours and about 1% of the p-chlorobenzotrifluoride had been converted to hydrodehalogenation by-product, trifluoromethylbenzene. The cooled reaction mixture was filtered and fractionated to yield 4-(trifluoromethyl)benzonitrile in 81% yield (boiling point 85-90 °C/28mm).

## Example VII

When in the above Example VI, an equal amount of acetonitrile is substituted for the t-butanol and the temperature for distilling off of azeotropic mixture is adjusted and the temperature used for catalyst formation and cyanation is 45 °C, and the cyanation is carried out over a period of 5 hours, 4-(trifluoromethyl)benzonitrile is still the major product and less than 1% hydrodehalogenation occurs. Similar results are obtained when tetrahydrofuran is substituted for the acetonitrile.

## Example VIII

A 100 ml flask is equipped with a Dean-Stark water separator and nitrogen atmosphere. The flask is charged with 0.26g (1.08 mmoles) of $NiCl_2 \cdot 6H_2O$ and 40 ml of toluene. The temperature is raised to 107 °C and the wet toluene azeotrope is distilled off and then the major portion of the waters of hydration are removed by azeotrope. The remainder of the toluene is distilled off under a positive nitrogen purge. Then the reactor is charged with triphenylphosphine (0.98g, 3.74 mmole), isopropyl alcohol (40 ml) and zinc powder (0.84 g, 12.8 mmole). The reaction is heated at 78 °C for 0.5 hours; 10.6 g. (58.7 mmole) of 4-chlorobenzotrifluoride is added and the reaction is again heated at 78 °C for 0.5 hours. Sodium cyanide (2.9 g; 59.2 mmole) is added and the reaction again heated at 78 °C until reaction stops. The 4-(trifluoromethyl)-benzonitrile is isolated by filtration of the reaction mixture followed by distillation of the product at 85 °C/28mm.

## Example IX

To a 250 mL round bottom flask was added $NiCl_2 \cdot 6H_2O$, (0.96 g, 4 mmoles) and the contents of the flask were heated between 80 - 100 °C for 0.25 hours under water aspirator vacuum to yield 0.56 g of essentially dry $NiCl_2$. The flask was now fitted with a thermometer, pressure equalizing addition funnel, and a Claisen head. To the Claisen head was attached a reflux condenser and a rubber stopper. The entire system was flushed with dry nitrogen via a gas inlet attached to the top of the condensor. The flask was now charged with acetonitrile , 80 mL, and triphenylphosphine, (4.2 g, 16 mmoles). p-Chlorobenzotrifluoride, (42.6 g, 25 mmoles) was charged to the addition funnel. The contents of the flask were sparged for 5 minutes using dry nitrogen and then warmed to 45 °C at which time the color of the contents indicated formation of $Ni(P\phi_3)_2Cl_2$. To the flask was now added zinc dust, (0.8 g, 8 mmoles) and the contents were stirred for 0.25 hours when the red color indicative of $Ni(P\phi_3)_3$ was observed. p-Chlorobenzotrifluoride, (42.6, 240 mmoles), was now added to the flask over 5 minutes followed by addition of sodium cyanide, (11.6 g, 240 mmoles). The reaction was followed by GC which indicated 80% conversion after 5 hours at 45 °C with 0.2% hydrodehalogenation by-product. The contents of the flask was cooled, filtered and the filtrate distilled to yield 76% 4-(trifluoromethyl)benzonitrile, bp. 85-90 °C/25 Torr.

When tetrahydrofuran is substituted for the acetonitrile in Example IX, 4-(trifluoromethyl)benzonitrile is the major product and there is less than 1% hydrodehalogenation .

When t-butanol is substituted for the acetonitrile and reflux temperature and a cyanation time of 18 hours are used, 4-(trifluoromethyl)benzonitrile is the major product and there is about 1% hydrodehalogenation.

## Example X

A 1L 3-necked flask fitted with a distillation head, thermometer and rubber stopper was charged with dry acetonitrile (320 ml), $NiCl_2 \cdot 6H_2O$ (0.96g, 4 mmoles), and $P\phi_3$ (4.2g, 16 mmoles). The contents of the flask was heated to reflux and was maintained at reflux so as to provide reaction to form wet $Ni(P\phi_3)_2Cl_2$ and to distill off 280 ml of distillate/azeotrope to provide substantially dry $Ni(P\phi_3)_2Cl_2$. To the now dark green contents of the flask were added 40 ml of dry acetonitrile. The contents were sparged with nitrogen for 5 minutes and then zinc dust (0.8g, 8 mmoles) was added in one portion. After stirring at 40-45 °C for 0.25 hours the contents of the flask changed in color from dark green to blood red. p-Chlorobenzotrifluoride (42.6g, 240 mmoles) and sodium cyanide (11.6g, 240 mmoles) were added in sequence as single portions. The reaction was monitored by GC which indicated 65% conversion after 5 hours with 0.4% hydrodehalogenation product.

When tetrahydrofuran is substituted for acetonitrile in Example X, similar results are obtained.

When t-butanol is substituted for the acetonitrile in Example X and complex formation is carried out by heating at reflux temperature for two hours and cyanation is carried out at reflux temperature for 18 hours, 4-(trifluoromethyl) benzonitrile is the major product and there is about 1% hydrodehalogenation.

When in Example X, the $NiCl_2 \cdot 6H_2O$ and $P\phi_3$ are reacted in the absence of acetonitrile to form wet $Ni(P\phi_3)_2Cl_2$ and then 320 ml of toluene is added and heating at reflux is carried out to distill off toluene azeotrope and the major portion of the water to form substantially dry $Ni(P\phi_3)_2Cl_2$ and then the rest of the toluene is distilled off and then 80 ml of acetonitrile is added to the substantially dry $Ni(P\phi_3)_2Cl_2$ instead of 40 ml, 4-(trifluoromethyl)benzonitrile is the major product and there is less than 1% hydrodehalogenation.

## Example XI

A 250 ml round bottom flask was fitted with a magnetic stirrer and thermometer. To the flask was added $NiCl_2 \cdot 6H_2O$, (0.96 g, 4 mmoles) and ($P\phi_3$, 4.2 g, 16 mmoles). The reactants were heated at 100 °C and the evolving moisture was flashed off in a stream of dry $N_2$. The reactor was then equipped with a reflux condenser. To the reaction mixture was added 80 ml of dry isopropyl alcohol and the solution was sparged with $N_2$ for 10 minutes. Powdered Zinc metal, (0.8 g, 8 mmoles) was added and the mixture heated at 77 °C for 2.25 hours, at which time the reaction mixture became red in color. 4-Chlorobenzotrifluoride (42.6 g, 240 mmoles) and sodium cyanide (11.6 g, 240 mmoles) were then added sequentially and the reaction was heated at reflux. The reaction was monitored via GLC which indicated 75% conversion after 5 hours. The extent of hydrodehalogenation product, i.e., trifluoromethylbenzene was 7.5% of the 4-chlorobenzotrifluoride reacted versus 76% for cyanation product,4-(trifluoromethyl)benzonitrile.

When t-butanol is substituted for the isopropanol in Example XI, 4-(trifluoromethyl)benzonitrile is the major product and there is about 1% hydrodehalogenation.

When acetonitrile is substituted for the isopropanol in Example XI and the temperature for the reaction to form $Ni(P\phi_3)_3$ and for cyanation is 45 °C, 4-(trifluoromethyl)benzonitrile is the major product and there is less than 1% hydrodehalogenation.

## Comparative Example I

A 100 mL flask was charged with $NiCl_2 \cdot 6H_2O$ (0.26g, 1.08 mmoles) and $P\phi_3$ (0.98g, 3.74 mmoles). The reaction mixture was heated to 65 °C (96 °C oil bath temperature) under a nitrogen purge during 0.5 hours. After cooling, 40 ml of isopropanol and zinc dust (0.84g, 12.8 mmoles) were added and the the reaction mixture was heated at 78 °C for 2.25 hours. p-Chlorobenzotrifluoride (10.6g, 58.7 mmoles) was added and the reaction mixture was heated at 55 °C for 1.25 hours. Sodium cyanide was added and the reaction mixture was heated at 56 °C for 6 hours. GLC assayed the liquid supernatant of the reaction mixture as:

p-chlorobenzotrifluoride, 53.0%; benzotrifluoride 14.6%; and 4-(trifluoromethyl)benzonitrile, 32.8%. The desired nitrile was isolated by filtration, followed by vacuum distillation, b.p. 85° C/28mm. The conversion of p-chlorobenzotrifluoride was 47% and 4-(trifluoromethyl)benzonitrile was 69% of that converted, and benzotrifluoride hydrodehalogenation product was 31% of that converted so the extent of hydrodehalogenation was 31%. This comparative example demonstrates that when wet reagents are used and not dried, the conversion is low and the percentage of hydrodehalogenation is increased.

Variations will be evident to those skilled in the art. Therefore, the scope of the invention is intended to be defined by the claims.

## Claims

1. A method of converting aromatic halide to aromatic nitrile utilizing $Ni(P\phi_3)_3$ as a catalyst, where said catalyst is formed starting with $NiCl_2$ or $NiCl_2 \cdot 6H_2O$ and where formation of said catalyst and conversion of halide to nitrile are carried out in a single reactor vessel, said aromatic halide having the formula:

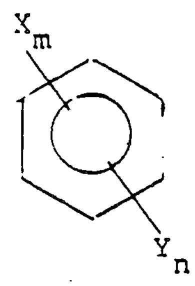

where X is
Cl or Br, Y is
CHO or $CF_3$, m ranges from 1 to 3, and n ranges from 0 to 2, said method comprising the steps of:

(a) forming said catalyst by steps comprising

(i) forming essentially dry $Ni(P\phi_3)_2Cl_2$ in situ in said reactor vessel starting with $NiCl_2$ or $NiCl_2 \cdot 6H_2O$ and $P\phi_3$;

(ii) reacting said $Ni(P\phi_3)_2Cl_2$ with at least one equivalent of $P\phi_3$ in the presence of a reducing metal with a greater reduction potential than nickel in a reaction solvent consisting of a $C_2$-$C_5$ alcohol or an aprotic polar solvent in said reactor vessel to produce $Ni(P\phi_3)_3$ catalyst therein;

(b) adding said aromatic halide into said reactor vessel to form a complex in said reaction solvent from said aromatic halide and said catalyst and adding NaCN or KCN into said reactor vessel to convert X to CN and thereby produce nitrile product.

2. The method of claim 1 wherein said aromatic halide is p-chlorobenzotrifluoride and the nitrile which is formed is 4-(trifluoromethyl)benzonitrile.

3. The method of any one of claims 1 to 2, wherein the reducing metal is zinc metal.

4. The method of any one of claims 1 to 3, wherein in step (a)(i) the essentially dry $Ni(P\phi_3)_2Cl_2$ is formed by steps comprising starting with $NiCl_2 \cdot 6H_2O$ and forming an admixture of $NiCl_2 \cdot 6H_2O$ in liquid which forms an azeotropic mixture with water and has a boiling point at least 10° C less than the ultimate nitrile product and heating to remove sufficient of the water of hydration to distill off sufficient water and sufficient of said liquid so as not to significantly interfere with reaction in steps (a)(ii) and (b) to form essentially dry $NiCl_2$, and reacting the formed essentially dry $NiCl_2$ with two equivalents of $P\phi_3$ in a reaction solvent selected from the group consisting of $C_2$-$C_5$ alcohols and aprotic polar solvents.

5. The method of any one of claims 1 to 3, wherein in step (a)(i) the essentially dry $Ni(P\phi_3)_2Cl_2$ is formed by steps comprising reacting $NiCl_2 \cdot 6H_2O$ and two equivalents of $P\phi_3$ to form wet $Ni(P\phi_3)_2Cl_2$ and drying by heating an admixture of the wet $Ni(P\phi_3)_2Cl_2$ with liquid which forms an azeotrope with water and has a boiling point at least 10° C less than the ultimate nitrile product to remove sufficient water and sufficient of said liquid so as not to significantly interfere with the reaction in steps (a)(ii) and (b).

6. A method as recited in claim 4 or claim 5 wherein the azeotrope-forming liquid in step (a)(i) is not a $C_2$-$C_5$ alcohol or an aprotic solvent.

7. A method as recited in claim 4 or claim 5 wherein the azeotrope-forming liquid in step (a)(i) is a $C_2$-$C_5$ alcohol or an aprotic polar solvent.

8. The method of any one of claims 1 to 5 and 7 wherein the azeotrope-forming liquid in step (a)(i) is t-butanol, acetonitrile or tetrahydrofuran.

9. The method of any one of claims 1 to 8, wherein the reaction solvent in steps (a)(ii) and (b) is t-butanol, acetonitrile or tetrahydrofuran.

10 . The method of any one of claims 1 to 3 wherein in step (a)(i) the essentially dry $Ni(P\phi_3)_2Cl_2$ is formed by steps comprising starting with $NiCl_2 \cdot 6H_2O$ and heating the $NiCl_2 \cdot 6H_2O$ with at least two equivalents of $P\phi_3$ in the absence of reaction solvent wherein water flashes off and essentially dry $Ni(P\phi_3)_2Cl_2$ is formed.

11 . The method of claim 10 wherein the reaction solvent in steps (a)(ii) and (b) is t-butanol or acetonitrile.

12. The method of any one of claims 1 to 3, wherein in step (a)(i) the essentially dry $Ni(P\phi_3)_2Cl_2$ is formed by steps ccmprising starting with anhydrous $NiCl_2$ and reacting the same with two equivalents of $P\phi_3$ in $C_2$-$C_5$ alcohol reaction solvent.

13. The method of claim 12, wherein the reaction solvent in steps (a)(i), (a)(ii) and (b) is isopropanol, t-butanol or 2-butanol.

14. The method of any one of claims 1 to 3, wherein in step (a)(i) the essentially dry $Ni(P\phi_3)_2Cl_2$ is formed by steps comprising starting with anhydrous $NiCl_2$ and reacting the same with two equivalents of $P\phi_3$ in an aprotic polar reaction solvent.

15. The method of claim 14 wherein the reaction solvent in steps (a)(i), (a)(ii) and (b) is acetonitrile or tetrahydrofuran.

16. The method of any one of claim 1 to 3, wherein in step (a)(i) the essentially dry $Ni(P\phi_3)_2Cl_2$ is formed by steps ccmprising starting with $NiCl_2 \cdot 6H_2O$ and heating in the absence of $P\phi_3$ and reaction solvent to form essentially dry $NiCl_2$ and reacting the essentially dry $NiCl_2$ with two equivalents of $P\phi_3$ in reaction solvent selected from the group consisting $C_2$-$C_5$ alcohols and aprotic polar solvents.

17. The method of claim 16 wherein the reaction solvent is acetonitrile or t-butanol.

European Patent Office

# EUROPEAN SEARCH REPORT

Application number

EP 90103235.9

## DOCUMENTS CONSIDERED TO BE RELEVANT

| Category | Citation of document with indication, where appropriate, of relevant passages | Relevant to claim | CLASSIFICATION OF THE APPLICATION (Int Cl 5) |
|---|---|---|---|
| A | US - A - 4 499 025 (J.B.DAVISON et al.) * Claims; examples * | 1,2,6, 7 | C 07 C 255/50<br>C 07 C 253/14 |
| A | CHEMICAL ABSTRACTS, vol. 82, no. 13, March 31, 1975, Columbus, Ohio, USA L.CASSAR et al. "Nickel-catalyzed cyanation of aromatic halides" page 465, column 2, abstract-no. 85 632y | 1 | |
| D | & Adv.chem.Ser., 1974,132, 252-73 | | |

TECHNICAL FIELDS SEARCHED (Int Cl 5)

C 07 C 253/00
C 07 C 255/00

The present search report has been drawn up for all claims

| Place of search | Date of completion of the search | Examiner |
|---|---|---|
| VIENNA | 15-05-1990 | HOFBAUER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document